# EUROPÄISCHE PATENTSCHRIFT

(11) **EP 1 818 006 B1**
(45) Veröffentlichungstag und Bekanntmachung des Hinweises auf die Patenterteilung: **11.01.2012**
(21) Anmeldenummer: 06405063.6
(22) Anmeldetag: 09.02.2006
(51) Int. Cl.: A61B 3/107, A61B 3/103

(54) **Ophthalmologische Messvorrichtung**
Ophthalmologic measurement device
Dispositif de mesure ophtalmologique

(43) Veröffentlichungstag der Anmeldung: 15.08.2007
(73) Patentinhaber: SIS AG, Surgical Instrument Systems, 2562 Port (CH)
(72) Erfinder: Rathjen, Christian, 28197 Bremen (DE)
(74) Vertreter: Vogel, Dany

(56) Entgegenhaltungen:
- WO-A-01/28410
- WO-A-2005/027741
- US-A- 5 512 965
- US-A1- 2002 163 623
- US-A1- 2004 119 943

## Beschreibung

### Technisches Gebiet

Die vorliegende Erfindung betrifft eine ophthalmologische Messvorrichtung mit einem Messsystem. Die vorliegende Erfindung betrifft insbesondere eine ophthalmologische Messvorrichtung mit einem Messsystem, das ein Lichtprojektionssystem umfasst, zur Projektion eines Lichtbündels durch einen Querschnittsteil eines Auges, sowie mit Bilderfassungsrnitteln, welche in Scheimpfluganordnung zum Lichtbündel angeordnet sind, zur Erfassung und Speicherung eines Querschnittsabbilds von mindestens einem Teilgebiet des durch das Lichtprojektionssystem beleuchteten Querschnittsteils.

### Stand der Technik

Im Stand der Technik sind ophthalmologische Messvorrichtungen und ophthalmologische Messverfahren bekannt, in welchen mittels eines Lichtprojektors ein Strahlenbündel durch einen Querschnittsteil eines Auges, insbesondere durch einen Querschnittsteil der Hornhaut, projiziert wird. Typischerweise wird das Strahlenbündel in Form eines Lichtspalts projiziert. In der Patentschrift US 5404884 werden ein Verfahren und eine Vorrichtung für die Untersuchung von Homhautgewebe eines Patienten beschrieben. Gemäss US 5404884 wird ein im wesentlichen ebener Laserstrahl mit einem spaltförmigen Profil durch einen Querschnittsteil der Hornhaut projiziert. Durch die Erfassung von mindestens einem Teil des in der Hornhaut gestreuten Lichts, das heisst von mindestens einem Teil des Lichtspalts, wird gemäss US 5404884 ein Querschnittsabbild der Hornhaut gewonnen. Aus mehreren solchen Querschnittsabbifdem der Hornhaut können gemäss US 5404884 Homhauttrübungen, Homhautdicke und Homhauttopografie umfassend für die gesamte Hornhaut bestimmt werden. Da sich die Augen relativ zu der Messvorrichtung bewegen können, kann die Untersuchung des gesamten Auges gemäss US 5404884 zu Ungenauigkeiten führen, weil diese Relativbewegungen nicht erfasst und berücksichtigt werden und weil sich beim Zusammenfügen der Querschnittsabbilder, bedingt durch die Schwierigkeit der gegenseitigen Ausrichtung, Messartefakte ergeben können.

In EP 1430829 wird eine ophthalmologische Messvorrichtung beschrieben, welche mittels eines Lichtprojektors ein Strahlenbündel (z.B. ein Lichtspalt) durch einen Querschnittsteil der Homhaut eines Auges projiziert. Die Vorrichtung nach EP 1430829 erfasst jeweils zusätzlich zum in Scheimpfluganordnung erfassten Querschnittsabbild des beleuchteten Querschnittsteils ein Ansichtabbild des Auges, das ein Abbild des beleuchteten Querschnittsteils umfasst. Auf der Basis des Ansichtabbilds bestimmt die Vorrichtung die relative Position des gespeicherten Querschnittsabbilds zum Auge und ermöglicht dadurch eine zusammenhängende Untersuchung des gesamten Auges (z.B. eine Topografie der Hornhaut), bei der Relativbewegungen des Auges zur Vorrichtung berücksichtigt werden.

Die Vorrichtung nach EP 1430829 ermöglicht die Erfassung von Messdaten für die Bestimmung geometrischer Augenparameter wie Hornhauttopografie oder Homhautdicke. Das Messsystem nach EP 1430829 liefert jedoch keine ausreichenden Daten für die Bestimmung von optischen Eigenschaften des Auges, insbesondere von optischen Eigenschaften der Augenlinse.

WO 01/28410 beschreibt ein System und ein Verfahren zum Vermessen der Hornhaut, in welchen Daten über die Hornhauttopografie mit erfassten Wellenfrontaberrationsdaten kombiniert werden. Zur Erfassung der Wellenfrontaberrationsdaten wird ein Messsystem mit einem Wellenfrontdetektor eingesetzt. Für die Bestimmung der Hornhauttopografie verweist WO 01/28410 auf die Patentschrift US 5,512,965, welche eine ophthalmologische Vorrichtung zur Vermessung der Hornhaut beschreibt. Gemäss US 5,512,965 werden in Scheimpfluganordnung mehrere Querschnittsabbilder der Hornhaut erfasst, die durch eine Lichtspaltlampe in mehreren Positionen durchleuchtet wird.

### Darstellung der Erfindung

Es ist eine Aufgabe der vorliegenden Erfindung eine ophthalmologische Messvorrichtung vorzuschlagen, welche ein Lichtprojektionssystem umfasst, zur Projektion eines Lichtbündels durch einen, Querschnittsteil eines Auges, sowie in Scheimpfluganordnung angeordnete Bilderfassungsmittel, zur Erfassung und Speicherung eines Querschnittsabbilds vom beleuchteten Querschnittsteil, welche nicht die Nachteile der bekannten ophthalmologischen Messvorrichtungen aufweist. Es ist insbesondere eine Aufgabe der vorliegenden Erfindung eine ophthalmologische Messvorrichtung vorzuschlagen, welche ermöglicht Messdaten zu erfassen für die Bestimmung von optischen Eigenschaften des Auges, insbesondere von optischen Eigenschaften der Augenlinse.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die ophthalmologische Messvorrichtung umfasst ein erstes Messsystem, welches ein erstes Lichtprojektionssystem umfasst, zur Projektion eines ersten Lichtbündels, beispielsweise in Form eines Lichtspalts, durch einen Querschnittsteil des Auges, und in Scheimpfluganordnung zum ersten Lichtbündel angeordnete Bilderfassungsmittel zur Erfassung und Speicherung eines Querschnittsabbilds von mindestens einem Teilgebiet des durch das erste Lichtprojektionssystem beleuchteten Querschnittsteils, aus einer ersten Position ausserhalb des ersten Lichtbündels.

Die oben genannten Ziele werden durch die vorliegende Erfindung insbesondere dadurch erreicht, dass die ophthalmologische Messvorrichtung ein zweites Messsystem umfasst, welches ein zweites Lichtprojektionssystem umfasst, zur Projektion eines zweiten Lichtbündels auf das Auge, und einen Wellenfrontdetektor zum Bestimmen und Speichern eines Wellenfrontverlaufs des durch das Auge reflektierten zweiten Lichtbündels. In verschiedenen Ausführungsvarianten umfassen das erste Lichtprojektionssystem und das zweite Lichtprojektionssystem eine gemeinsame Lichtquelle oder eigene Lichtquellen mit vorzugsweise unterschiedlichen Wellenlängen. Das zweite Messsystem ermöglicht die Erfassung erweiterter Messdaten, welche zusätzlich zu Querschnittsabbildern auch den Wellenfrontverlauf umfassen, so dass basierend auf den erfassten Messdaten nicht nur geometrische Augenparameter, sondern auch optische Eigenschaften des Auges bestimmt werden können, insbesondere optische Eigenschaften der Augenlinse. Durch die in Scheimpfluganordnung erfassten Querschnittsabbilder von beleuchteten Querschnittsteilen des Auges können Augenstrukturen wie Augenhomhaut und Augenlinse geometrisch erfasst werden. Durch die Erfassung des Wellenfrontverlaufs des vom Auge reflektierten Lichtstrahlenbündels können die Wellenaberration und Brechkraft des Auges bestimmt werden. Die Kombination der beiden Systeme ermöglicht die Bestimmung eines verbesserten Augenmodells, in welchem insbesondere die Geometrie (Topografie) und optischen Eigenschaften der Augenlinse (Brechkraft) genauer definiert sind. Insbesondere kann die Bestimmung der Geometrie der Augenlinse, welche durch die in Scheimpfluganordnung leicht verzerrt erfassten Querschnittsabbilder beeinträchtigt ist, durch die aus dem Wellenfrontverlauf gewonnen Zusatzinformationen verbessert werden. Eine genauere Bestimmung der Geometrie der Augenlinse ist insbesondere bei der Wahl von lntraokularlinsen bei Katarakt (grauer Star) von Vorteil. Als weitere optische Eigenschaft der Augenlinse können auch Trübungen der Augenlinse im Querschnittsabbild bestimmt werden.

Vorzugsweise sind das erste Lichtprojektionssystem und das zweite Lichtprojektionssystem so angeordnet, dass das erste Lichtbündel und das zweite Lichtbündel entlang einer gemeinsamen Strahlungsachse auf das Auge projiziert werden. Die Strahlungsachse ist vorzugsweise die Sehachse oder, in einer Alternative, die visuelle (oder optische) Achse des Auges.

Vorzugsweise ist das erste Messsystem eingerichtet, das erste Lichtbündel durch mehrere verschieden positionierte Querschnittsteile des Auges zu projizieren und mehrere Querschnittsabbilder von jeweils mindestens einem Teilgebiet dieser beleuchteten Querschnittsteile in Scheimpfluganordnung zu erfassen und zu speichern. Die Messvorrichtung umfasst insbesondere ein Antriebsmodul zum Rotieren des ersten Messsystems im wesentlichen um eine Normale zu der dem ersten Lichtprojektionssystem zugewandten Oberfläche des Auges oder zum Verschieben des ersten Messsystems im wesentlichen senkrecht zu dieser Normalen. Die Normale fällt vorzugsweise mit der Sehachse des Auges oder, in einer Alternative, mit der visuellen (optischen) Achse des Auges zusammen. Durch das Antriebsmodul wird eine automatisierte zusammenhängende Untersuchung des gesamten Auges basierend auf mehreren Querschnittsabbildern ermöglicht. Die Messvorrichtung umfasst beispielsweise Verarbeitungsmittel, welche eingerichtet sind zum Bestimmen einer Homhauttopografie des Auges basierend auf den Querschnittsabbildem.

In einer Ausführungsvariante umfasst die Messvorrichtung ein drittes Messsystem, welches einen Schirmkörper mit einem sichtbaren Muster umfasst, wobei der Schirmkörper so angeordnet ist, dass das sichtbare Muster bei der Applikation der Messvorrichtung auf einer dem Auge zugewandten Seite des Schirmkörpers liegt. Das dritte Messsystem umfasst überdies Bilderfassungsmittel zur Erfassung und Speicherung eines Spiegelbilds des Musters auf dem Auge. Der Schirmkörper ist vorzugsweise so eingerichtet und angeordnet, dass das erste Lichtbündel ungehindert durch den Querschnittsteil des Auges projizierbar ist, dass das Querschnittsabbild ungehindert erfassbar ist, dass das zweite Lichtbündel ungehindert auf das Auge projizierbar ist, und dass das durch das Auge reflektierte zweite Lichtbündel ungehindert erfassbar ist. Das dritte Messsystem ermöglicht die Erfassung erweiterter Messdaten, welche zusätzlich zu den Querschnittsabbildern und dem Wellenfrontverlauf das erfasste Spiegelbild des Musters umfassen.

In einer Ausführungsvariante umfasst die Messvorrichtung Verarbeitungsmittel, welche eingerichtet sind zum Bestimmen einer musterbasierten Homhauttopografie des Auges basierend auf dem Spiegelbild, zum Bestimmen einer querschnittbasierten Homhauttopografie des Auges basierend auf den Querschnittsabbildem, und zum Bestimmen einer kombinierten Homhauttopografie des Auges basierend auf der musterbasierten Homhauttopografie und der querschnittbasierten Homhauttopografie. Die Querschnittbilder und das erfasste Spiegelbild des Musters werden so bei der Bestimmung der Topografie der Hornhaut einander ergänzend verwendet, beispielsweise als Kompensation von Messdaten, die auf Grund von Wimpemschlägen oder anderen Schatten unvollständig sind. An dieser Stelle soll angeführt werden, dass im vorliegenden Text mit dem Begriff Topografie sowohl das dreidimensionale Profil als auch das Neigungsprofil von Augenstrukturen wie Hornhaut, Linse etc. gemeint ist.

Vorzugsweise umfasst die Messvorrichtung ein Steuermodul, das eingerichtet ist zum wahlweisen Aktivieren von mindestens einem aus dem ersten Messsystem, dem zweiten Messsystem und dem dritten Messsystem zur Erfassung von mindestens einem aus dem Wellenfrontverlauf, dem Querschnittsabbild und dem Spiegelbild. Das Steuermodul ist beispielsweise eingerichtet eines oder mehrere der Messsysteme entsprechend einem benutzerselektierten Operationsmodus zur Erfassung von Wellenfrontverlauf, Spiegelbild und/oder Querschnittsabbild(em) zu aktivieren. Zudem umfasst die Messvorrichtung Verarbeitungsmittel, welche eingerichtet sind entsprechend dem benutzerselektierten Operationsmodus mindestens eine Augencharakteristik zu bestimmen basierend auf dem Wellenfrontverlauf, dem Spiegelbild und/oder dem/den Querschnittsabbild(em). Durch die wahlweise Aktivierung kann die Funktionalität der Messvorrichtung flexibel an die aktuellen Bedürfnisse des Benutzers angepasst werden und die Messsysteme können einzeln oder kombiniert zur Erfassung der verschiedenen Messdaten aktiviert werden. Entsprechend der Definition und Konfiguration verschiedener Operationsmodi, beispielsweise für verschiedene Messgrössen und/oder Messverfahren, können die benötigten Messsysteme automatisch aktiviert und die dadurch erfassten Messdaten durch die Verarbeitungsmittel automatisch ausgewertet werden.

In einer Ausführungsvariante ist das Steuermodul eingerichtet, mehrere der Messsysteme entsprechend dem benutzerselektierten Operationsmodus in einer definierten Reihenfolge nacheinander zur Erfassung von Wellenfrontverlauf, Spiegelbild und/oder Querschnittsabbild(em) zu aktivieren. Vorzugsweise werden die Messdaten jedoch durch die Messsysteme gleichzeitig erfasst.

Die Verarbeitungsmittel sind vorzugsweise eingerichtet zum Bestimmen von geometrischen und/oder optischen Eigenschaften der Augenlinse basierend auf den Querschnittsabbildern und dem Wellenfrontverlauf. Die Verarbeitungsmittel sind insbesondere eingerichtet zum Bestimmen der Topografie und/oder Brechkraft der Linse basierend auf den Querschnittsabbildern und dem Wellenfrontverlauf. Die Verarbeitungsmittel sind beispielsweise eingerichtet zum Bestimmen einer Wellenaberration des Auges basierend auf dem Wellenfrontverlauf, zum Bestimmen einer Homhauttopografie des Auges basierend auf den Querschnittsabbildem, und zum Bestimmen der Brechkraft der Linse basierend auf der Wellenaberration und der Homhauttopografie.

In einer Ausführungsvariante umfasst die Messvorrichtung weitere Bilderfassungsmittel zur Erfassung eines Ansichtabbilds des Auges, welches Ansichtabbild ein Abbild des durch den ersten Lichtprojektor beleuchteten Querschnittsteiis und/oder das Spiegelbild des Musters auf dem Auge umfasst, und zur Speicherung dieses Ansichtabbilds zugeordnet zum Querschnittsabbild. Zudem umfasst die Messvorrichtung Verarbeitungsmittel zur Positionierung des gespeicherten Querschnittsabbilds relativ zum Auge auf der Basis des zugeordnet gespeicherten Ansichtabbilds. Die Erfassung und Speicherung des Querschnittsabbilds und des dazu gehörenden Ansichtabbilds mit dem beleuchteten Querschnittsteil ermöglicht die Bestimmung der Position des Querschnittsabbilds respektive des darin erfassten beleuchteten Querschnittsteils relativ zum Auge auf der Basis des zugeordneten Ansichtabbilds des durch den ersten Lichtprojektor beleuchteten Querschnittsteils und/oder des Spiegelbilds des Musters auf dem Auge.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
Figur 1 zeigt ein Blockdiagramm, welches schematisch eine ophthalmologische Messvorrichtung illustriert, welche mehrere verschiedene Messsysteme, eine Anzeige sowie Verarbeitungsmittel mit einem Steuermodul umfasst.
Figur 2 zeigt ein Blockdiagramm, welches schematisch ein erstes Messsystem der ophthalmologischen Messvorrichtung illustriert, das ein erstes Lichtprojektionssystem, eine Bilderfassungsvorrichtung zur Erfassung eines Querschnittsabbilds eines Auges sowie eine optionale Bilderfassungsvorrichtung zur Erfassung eines Ansichtabbilds des Auges umfasst.
Figur 3 zeigt ein Blockdiagramm, welches schematisch ein zweites Messsystem der ophthalmologischen Messvorrichtung illustriert, das ein zweites Lichtprojektionssystem sowie einen Wellenfrontdetektor umfasst.
Figur 4 zeigt ein Blockdiagramm, welches schematisch ein drittes Messsystem der ophthalmologischen Messvorrichtung illustriert, das eine Bilderfassungsvorrichtung zur Erfassung eines Ansichtabbilds des Auges sowie einen durchbrochenen Schirmkörper umfasst.
Figur 5 zeigt ein Blockdiagramm, welches schematisch eine ophthalmologische Messvorrichtung illustriert, die ein erstes und ein zweites Messsystem umfasst.
Figur 6 zeigt ein Blockdiagramm, welches schematisch eine ophthalmologische Messvorrichtung illustriert, die ein erstes und ein zweites Messsystem umfasst, wobei der Lichtprojektor des zweiten Messsystems Teil des Lichtprojektionssystems des ersten Messsystems ist.
Figur 7 zeigt ein Blockdiagramm, welches schematisch eine ophthalmologische Messvorrichtung illustriert, die ein erstes und ein zweites Messsystem umfasst, wobei das erste Messsystem einen Lichtprojektor und Bilderfassungsmittel zur Erfassung zweier Querschnittsabbilder und eines Ansichtabbilds eines Auges umfasst.
Figur 8 zeigt ein Blockdiagramm, welches schematisch eine ophthalmologische Messvorrichtung illustriert, die ein erstes und ein zweites Messsystem umfasst, wobei das erste Messsystem einen Lichtprojektor und Bilderfassungsmittel zur Erfassung zweier Querschnittsabbilder und eines Ansichtabbilds eines Auges umfasst, und wobei der Wellenfrontdetektor des zweiten Messsystems Teil eines Bildwandlers des ersten Messsystems ist.
Figur 9 zeigt ein Blockdiagramm, welches schematisch eine ophthalmologische Messvorrichtung illustriert, die ein erstes, ein zweites und ein drittes Messsystem umfasst.
Figur 10 zeigt ein Blockdiagramm, welches schematisch eine ophthalmologische Messvorrichtung illustriert, die ein erstes, ein zweites und ein drittes Messsystem umfasst, wobei der Lichtprojektor des zweiten Messsystems Teil des Lichtprojektionssystems des ersten Messsystems ist.
Figur 11 zeigt eine Ansicht der dem Auge zugewandten Seite eines Schirmkörpers mit Durchbrechungen und einem sichtbaren Muster.
Figur 12a zeigt ein Querschnittsabbild eines beleuchteten Querschnittsteils eines Auges (Hornhaut).
Figur 12b zeigt ein Ansichtabbild eines Auges mit einem beleuchteten Querschnittsteil.
Figur 13 zeigt ein kombiniertes Abbild mit einem Querschnittsabbild eines beleuchteten Querschnittsteils eines Auges und einem Ansichtabbild des Auges mit dem beleuchteten Querschnittsteil.

### Wege zur Ausführung der Erfindung

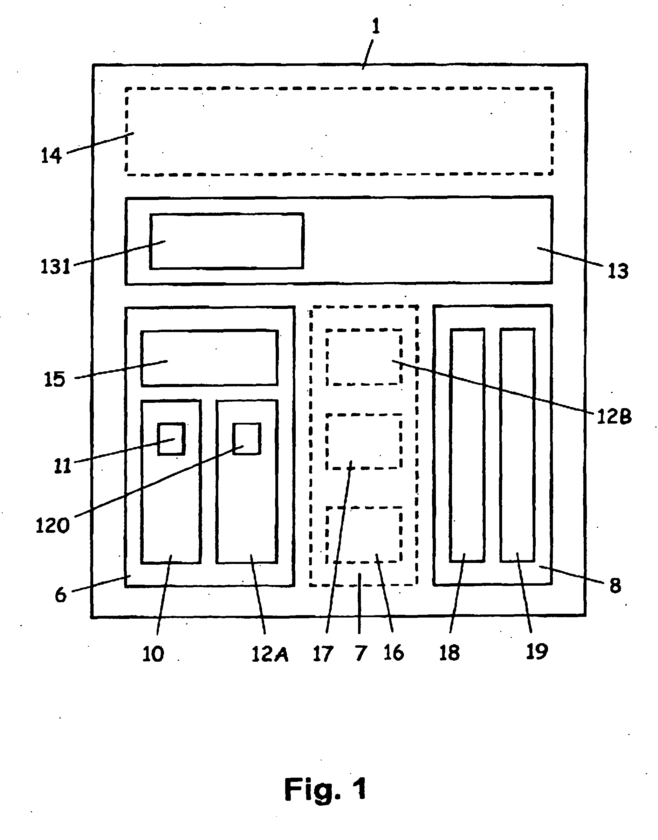

In den Figuren 1, 5, 6, 7, 8, 9, 10 bezeichnet das Bezugszeichen 1 eine ophthalmologische Messvorrichtung, wobei in der nachfolgenden Beschreibung mit Bezug auf diese Figuren verschiedene Ausführungsformen der ophthalmologischen Messvorrichtung 1 erläutert werden. Ansonsten werden in den Figuren einander entsprechende, gleiche Komponenten durch gleiche Bezugszeichen bezeichnet.

Wie in der Figur 1 dargestellt ist, umfasst die ophthalmologische Messvorrichtung 1, ein erstes Messsystem 6, mit einem ersten Lichtprojektionssystem 10 und Bilderfassungsmitteln 12A, ein zweites Messsystem 8, mit einem zweiten Lichtprojektionssystem 18 und einem Wellenfrontdetektor 19, sowie ein optionales drittes Messsystem 7, mit Bilderfassungsmitteln 12B, einer zusätzlichen Lichtquelle 16 und einem Schirmkörper 17. Vorzugsweise werden die Bilderfassungsmittel 12B zur Erfassung von Ansichtabbildern vom ersten und dritten Messsystem 6, 7 gemeinsam verwendet. Das erste Lichtprojektionssystem 10 umfasst einen Lichtprojektor 11. Die Bilderfassungsmittel 12A umfassen einen Bildwandler 120. Die ophthalmologische Messvorrichtung 1 umfasst zudem nicht dargestellte Bedienungselemente, eine optionale Anzeige 14, auf welcher bestimmte Messwerte und/oder Applikationshilfen angezeigt werden, sowie Verarbeitungsmittel 13, mit einem Steuermodul 131 und weiteren funktionalen Modulen zur Verarbeitung von Messdaten, die von den Messsystemen 6, 7, 8 erfasst werden. Die Verarbeitungsmittel 13 umfassen mindestens einen Prozessor, Daten- und Programmspeicher. Die funktionalen Module sind vorzugsweise als programmierte Softwaremodule ausgeführt, die im Programmspeicher gespeichert sind und auf dem Prozessor ausgeführt werden. Der Fachmann wird verstehen, dass die funktionalen Module auch teilweise oder vollständig hardwaremässig ausgeführt werden können. Die elektrische Speisung der ophthalmologischen Messvorrichtung 1 erfolgt durch eine interne oder durch eine mittels Kabel angeschlossene externe Energiequelle.

An dieser Stelle soll festgehalten werden, dass die ophthalmologische Messvorrichtung 1 in einer Ausführungsvariante nur zur Erfassung der Messdaten, die von den Messsystemen 6, 7, 8 erfasst werden, eingerichtet ist und eine Geräteschnittstelle umfasst zur Übermittlung dieser Messdaten an funktionale Module, die extern zur ophthalmologischen Messvorricfirtung 1 angeordnet sind.

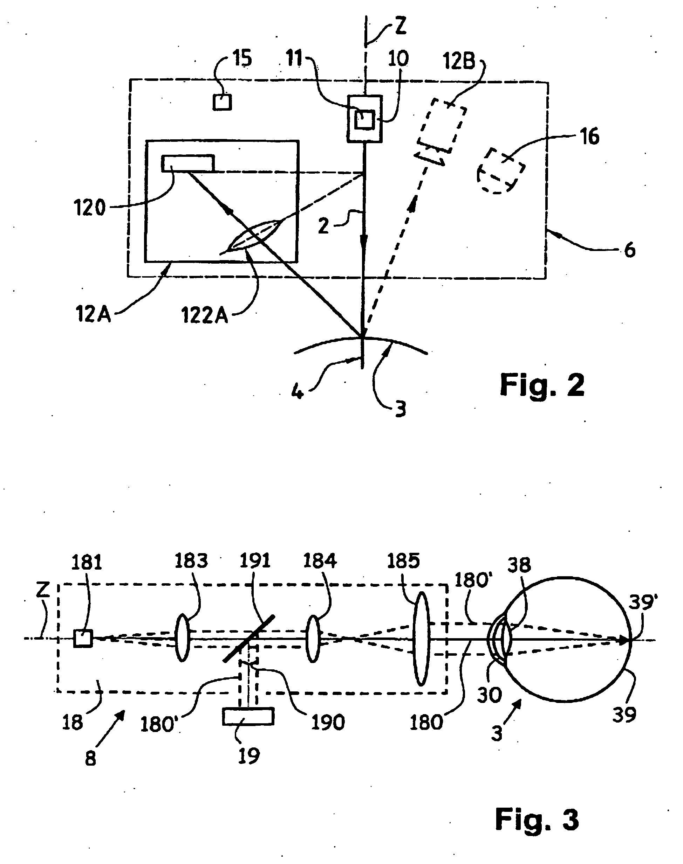

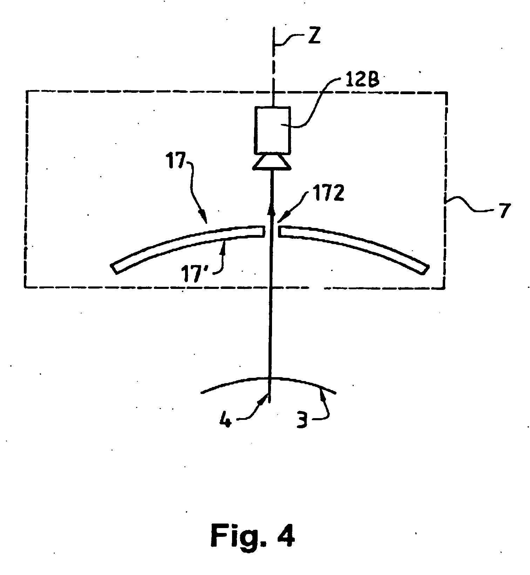

Eine Ausführung des ersten Messsystems 6 ist in der Figur 2 detailliert dargestellt. Die Figur 3 zeigt eine detaillierte Darstellung einer Ausführung des zweiten Messsystems 8 und die Figur 4 zeigt eine detaillierte Darstellung des dritten Messsystems 7.

Wie in den Figuren 2, 5, 6, 7, 8, 9, 10 schematisch dargestellt ist, ist das (erste) Lichtprojektionssystem 10 respektive der Lichtprojektor 11 eingerichtet zur Projektion eines Strahlenbündels 2 durch einen Querschnittsteil 4 des Auges 3, insbesondere durch einen Querschnittsteil 4 der Hornhaut 30 (Cornea) des Auges 3. Das Strahlenbündel 2 wird vorzugsweise in der Form eines Lichtspalts projiziert. Das Lichtprojektionssystem 10 respektive der Lichtprojektor 11 umfasst beispielsweise eine Spaltlampe oder einen Laser, dessen Licht durch Strahlumformungsoptiken zu einem Fächer geformt wird.

Wie in den Figuren 2, 5, 6, 7, 8, 9, 10 dargestellt ist, umfasst das erste Messsystem 6 respektive die ophthalmologische Messvorrichtung 1 zudem Bilderfassungsmittel zur Erfassung und Speicherung eines Querschnittsabbilds 30A von mindestens einem Teilgebiet des durch den Lichtprojektor 11 beleuchteten Querschnittsteils 4, welche in Scheimpfluganordnung zum Strahlenbündel 2 angeordnet sind.

Die in den Figuren 2, 5, 6, 7, 8, 9, 10 dargestellten Ausführungsformen des ersten Messsystems 6 respektive der ophthalmologischen Messvorrichtung 1 umfassen zudem weitere Bilderfassungsmittel zur Erfassung eines Ansichtabbilds 3A des Auges 3, welches ein Abbild des beleuchteten Querschnittsteils 4A umfasst, und zur Speicherung des erfassten Ansichtabbilds 3A und des darin enthaltenen Abbilds des beleuchteten Querschnittsteils 4A zugeordnet zum erfassten Querschnittsabbild 30A.

Wie in den Figuren 2, 5, 6, 7, 8, 9, 10 dargestellt ist, umfassen die Bilderfassungsmittel je nach Ausführungsform des ersten Messsystems 6 respektive der ophthalmologischen Messvorrichtung 1 Bilderfassungsvorrichtungen 12A, 12B, zum Beispiel CCD-Kameras (Charged Coupled Device) oder CMOS-Kameras (Complementary Metal-Oxide-Silicon), Bildwandler 120, zum Beispiel CCD-Chips oder CMOS-Chips, strahlumlenkende optische Elemente 121A, 121B, zum Beispiel Spiegel, und/oder abbildende optische Elemente 122A, 122B, zum Beispiel optische Linsen.

Zur Sichtbarmachung von natürlichen Augenmerkmalen, wie Limbus 33, Iris 34 oder Pupille 35, und/oder zur Projektion von künstlichen Lichtmarken 36 umfassen die in den Figuren 2, 5, 6, 7, 8, 9, 10 dargestellten Ausführungsformen des ersten Messsystems 6 respektive der ophthalmologischen Messvorrichtung 1 eine oder mehrere zusätzliche Lichtquellen 16. Insbesondere zur Sichtbarmachung natürlicher Augenmerkmale können beispielsweise eine oder mehrere Infrarotleuchtdioden verwendet werden. Die natürlichen und/oder künstlichen Referenzmerkmale werden im Ansichtabbild 3A des Auges 3 miterfasst.

Obwohl dies in den Figuren zur Vereinfachung nicht dargestellt ist, umfasst die ophthalmologische Messvorrichtung 1 ein Fixiertarget um das Auge 3 im akkomodierten und/oder unakkornodierten Zustand zu fixieren (feste Ausrichtung und Brechkraft des Auges 3). Das Fixiertarget ist beispielsweise ein Bild, das dem Patienten mittels eines weiteren Lichtstrahlenbündels in das Auge 3 projiziert wird.

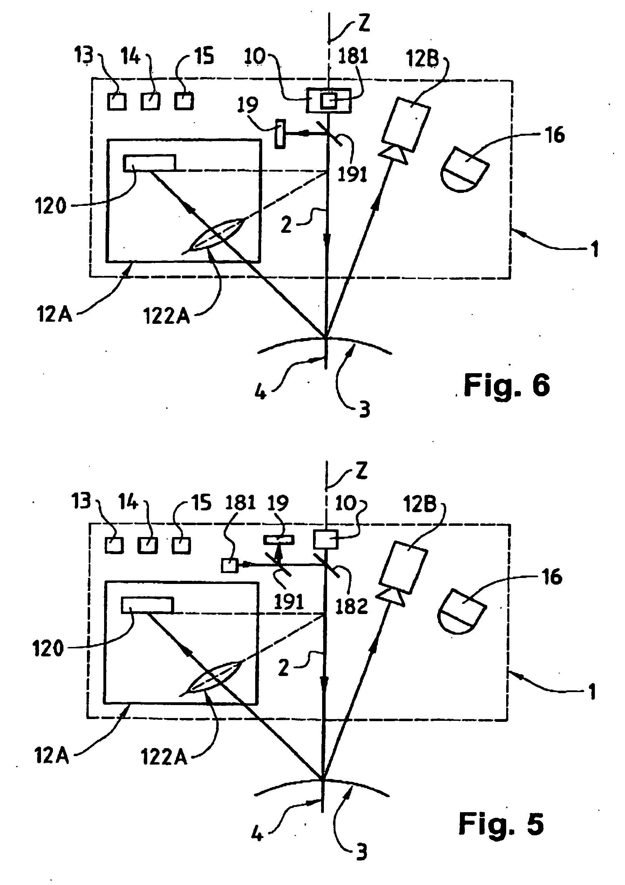

Wie in der Figur 3 schematisch dargestellt ist, ist das (zweite) Lichtprojektionssystem 18 respektive der Lichtprojektor 181 des zweiten Messsystems 8 eingerichtet zur Projektion eines Strahlenbündels 180 durch die Hornhaut 30 und die Augenlinse 38 auf die Retina (Netzhaut) 39 des Auges 3. Das Strahlenbündel 180 wird vorzugsweise als feiner Strahl ausgebildet und projiziert einen annähernd punktförmigen Fleck 39' auf der Retina 39, beispielsweise durch die abbildenden optischen Elemente 183, 184, 185, z.B. optische Linsen. Das Strahlenbündel 180 ist beispielsweise parallelgerichtet. Zur Fokussierung des Strahlenbündels 180 auf der Retina 39 umfasst das Lichtprojektionssystem 18 beispielsweise überdies ein nicht dargestelltes herkömmliches optisches Zoomsystem oder eine Linsenanordnung. Das von der Retina 39 diffus zurückgestreute Lichtbündel 180' fällt durch die Augenlinse 38 und die Hornhaut 30 auf das Lichtprojektionssystem 18 zurück, wo es zur Bestimmung des Wellentrontverlaufs 190 dem Wellenfrontdetektor 19 zugeführt wird, beispielsweise durch die abbildenden optischen Elemente 185, 184, z.B. optische Linsen, und das strahlumlenkende optische Element 191, z.B. ein halbdurchlässiger Spiegel. In den Ausführungsvarianten nach 5, 7, 9 wird das diffus zurückgestreute Lichtbündel 180' dem Wellenfrontdetektor 19 über mehrere strahlumlenkende optische Elemente 182, 191 zugeführt. In den Figuren 5. 6, 7, 8, 9, 10 wurden die Strahlumformungselemente (Linsen) zur Vereinfachung weggelassen. Der Wellenfrontdetektor 19 ist beispielsweise als Shack-Hartmann-Sensor ausgeführt, beispielsweise nach US 2003/0038921, oder als Interferometer, z.B. als Shearing-Interferometer. Weitere mögliche Ausführungsformen des Wellenfrontdetektors 19 sind in Jos. J. Rozena, Dirk E.M. Van Dyck, Marie-Jose Tassignon, "Clinical comparison of 6 aberrometers. Part 1: Technical specifications", J Cataract Refract Surg, Volume 31, Juni 2005, Seiten 1114-1127, beschrieben.

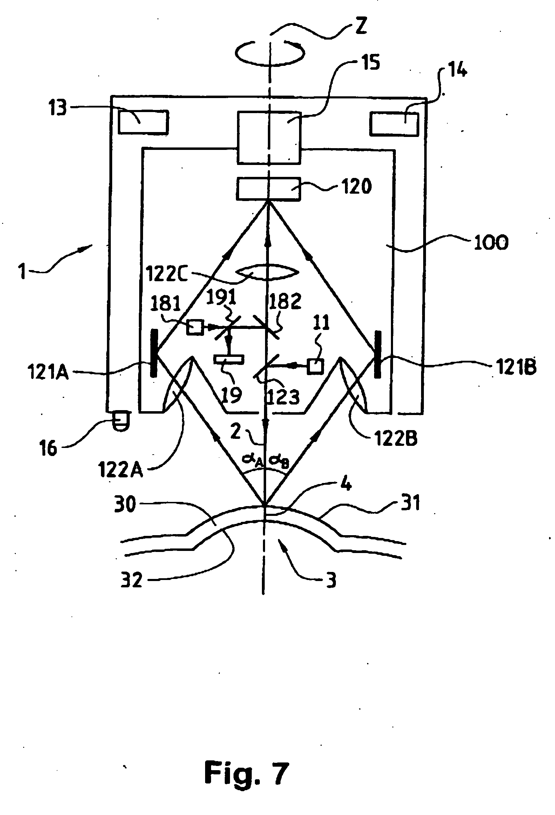

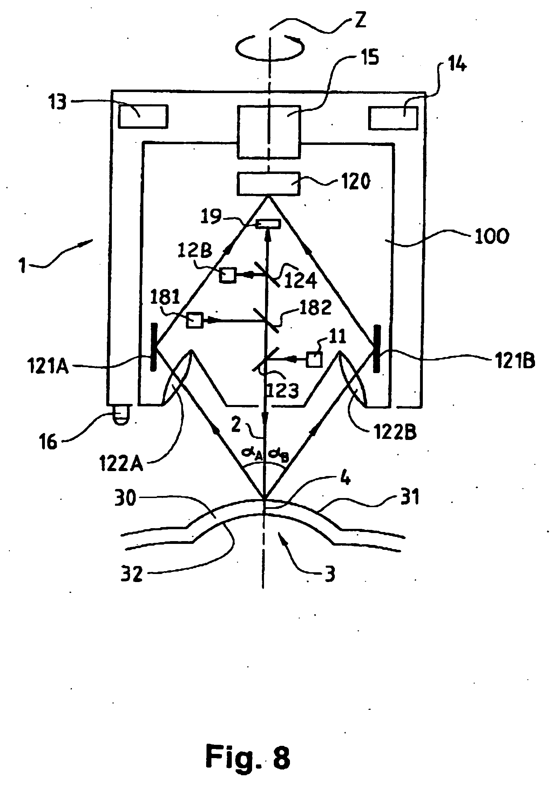

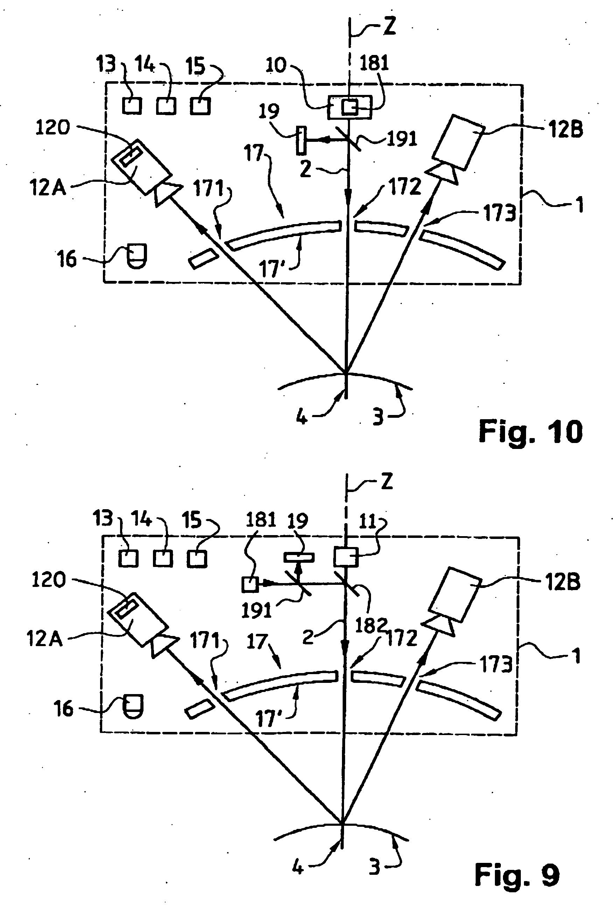

Wie in den Figuren 5, 6, 7, 8, 9, 10 dargestellt ist, sind die Lichtprojektionssysteme 10, 18 respektive die Lichtprojektoren 11, 181 vorzugsweise so angeordnet, dass die Lichtbündel 2, 180 entlang einer gemeinsamen Strahlungsachse Z auf das Auge 3 projiziert werden, vorzugsweise entlang der Sehachse des Auges 3, welche durch die Fovea und das Zentrum der Pupille 35 (sowie gegebenenfalls durch das Fixiertarget) definiert ist, oder durch die visuelle (oder optische) Achse des Auges 3. Wie in den Figuren 5, 7, 8, 9 dargestellt ist, umfasst die ophthalmologische Messvorrichtung 1 in verschiedenen Ausführungsvarianten ein strahlumlenkendes optisches Element 182, z.B. ein halbdurchlässiger Spiegel, zum Einleiten des Strahlenbündels 180 auf die Strahlungsachse Z. In alternativen Ausführungsvarianten ist der Lichtprojektor 181, wie in den Figuren 6 und 10 dargestellt ist, im Lichtprojektionssystem 10 angeordnet. Das heisst, das vom Lichtprojektor 181 des zweiten Messsystems 8 abgestrahlte Lichtbündel 180 wird über optische Elemente, z.B. abbildende und/oder strahlumlenkende optische Elemente, des Lichtprojektionssystems 10 des ersten Messsystems 6 entlang der Strahlungsachse Z auf das Auge 3 projiziert. In den Ausführungsvarianten nach den Figuren 7, 8 wird das vom Lichtprojektor 11 abgestrahlte Strahlenbündel 2 über ein strahlumlenkendes optisches Element 123, z.B. ein halbdurchlässiger Spiegel, auf die Strahlungsachse Z geleitet. In einer Ausführungsvariante werden die Lichtprojektoren 11, 181 von einer gemeinsamen Lichtquelle gespeist, z.B. ein Laser. Vorzugsweise umfassen die Lichtprojektoren 11, 181 jedoch ihre eigenen Lichtquellen mit jeweils unterschiedlichen Wellenlängen. In einer Ausführungsvariante umfasst der Lichtprojektor 11 eine Lichtquelle zur Erzeugung von Licht im blauen Bereich (Blaulichtquelle) und der Lichtprojektor 181 umfasst eine Lichtquelle zur Erzeugung von Licht im Infrarotbereich (Infrarotlichtquelle).

In den Ausführungen nach den Figuren 5, 6, 7, 8 umfasst die ophthalmologische Messvorrichtung 1 eine Kombination des ersten Messsystems 6 und des zweiten Messsystems 8. In den Ausführungen nach den Figuren 9, 10 umfasst die ophthalmologische Messvorrichtung 1 eine Kombination des ersten Messsystems 6, des zweiten Messsystems 8 und des dritten Messsystems 7.

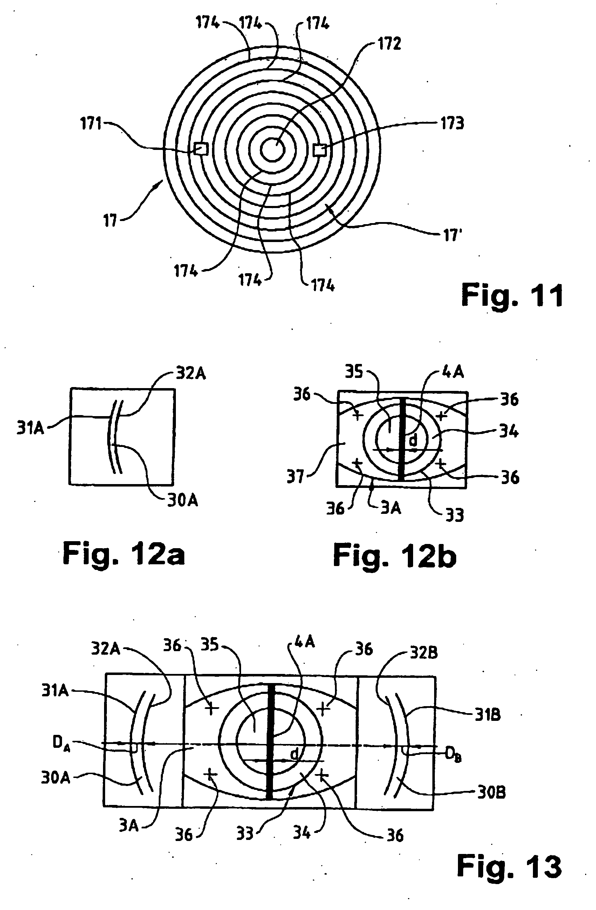

In den Ausführungsformen nach den Figuren 5 und 6 umfassen die Bilderfassungsmittel 12A zur Erfassung und Speicherung von Querschnittsabbildern 30A abbildende optische Elemente 122A und einen Bildwandler 120, welche in Scheimpfluganordnung zum projizierten Strahlenbündel 2 angeordnet sind. In der Figur 12a ist ein durch die Bilderfassungsvorrichtung 12A erfasstes Querschnittsabbild 30A des beleuchteten Querschnittsteils 4 des Auges 3 dargestellt. Im Querschnittsabbild 30A sind insbesondere ein Querschnittsabbild der vorderen Homhautoberfläche 31A und ein Querschnittsabbild der hinteren Homhautoberfläche 32A sichtbar. Obwohl dies nicht dargestellt ist, umfasst das Querschnittsabbild 30A zudem ein Querschnittsabbild der vorderen Linsenoberfläche und ein Querschnittsabbild der hinteren Linsenoberfläche. Die optische Achse der separaten Bilderfassungsvorrichtung 12B zur Erfassung des Ansichtabbilds 3A des Auges 3 befindet sich ausserhalb des Strahlenbündels 2. Das in der Figur 12b dargestellte Ansichtabbild 3A des Auges 3 entspricht allerdings einem Ansichtabbild, das durch eine Bilderfassungsvorrichtung 12B als Aufsicht erfasst wird, wobei die Bilderfassungsvorrichtung 12B so angeordnet ist, dass ihre optische Achse im Wesentlichen parallel zu der optischen Achse des Auges 3 verläuft oder, bevorzugt, mit der Strahlungsachse Z zusammenfällt. Im Ansichtabbild 3A sind insbesondere ein Abbild des beleuchteten Querschnittsteils 4A mit der endlichen Dicke d, die projizierten Lichtmarken 36 sowie Limbus 33, Iris 34 und Pupille 35 des Auges 3 sichtbar. Lichtmarken sind beispielsweise Glanzlichter von Leuchtdioden oder aufprojizierte Punkte. Projektionsorte sind beispielsweise die Sklera 37 oder die Homhaut 30.

In den Ausführungsformen nach den Figuren 7 und 8 weist die ophthalmologische Messvorrichtung 1 eine bevorzugte Anordnung auf, in welcher die Strahlungsachse Z und die optische Achse der Bilderfassungsmittel zur Erfassung des Ansichtabbilds 3A zusammenfallen. Die Bilderfassungsmittel umfassen einen gemeinsamen Bildwandler 120 zur Erfassung von zwei Querschnittsabbildern 30A, 30B aus zwei verschiedenen Positionen sowie nach Figur 7 auch zur Erfassung des Ansichtabbilds 3A. Die abbildenden optischen Elemente 122A und das strahlumlenkende optische Element 121A leiten die Lichtstrahlen zur Erfassung des Querschnittsabbilds 30A aus einer ersten Position unter dem Beobachtungswinkel α_{A} zur Erfassung an den Bildwandler 120. Die zusätzlichen abbildenden optischen Elemente 122B und das zusätzliche strahlumlenkende optische Element 121B leiten die Lichtstrahlen zur Erfassung des Querschnittsabbilds 30B aus einer zweiten Position unter dem BeobachtungsvAnkel α_{B} zur Erfassung ebenfalls an den Bildwandler 120. Vorzugsweise befinden sich die beiden Positionen auf verschiedenen Seiten des Strahlenbündels 2 und die Beträge der Beobachtungswinkel α_{A} und α_{B} sind vorzugsweise gleich gross. Nach Figur 8 werden die Lichtstrahlen zur Erfassung des Ansichtabbilds 3A über ein (wellenlängenselektives) strahlumlenkendes optisches Element 124 zur Bilderfassungsvorrichtung 12B geleitet. Die Figur 13 zeigt eine mögliche Kombination von Querschnittsabbild 30A, Ansichtabbild 3A und Querschnittsabbild 30B, welche durch den Bildwandler 120 der Ausführungsformen gemäss der Figur 7 erfasst wird. Die funktionalen Module der Verarbeitungsmittel 13 umfassen programmierte Auswertungsmodule, welche in den erfassten und gespeicherten Querschnittsabbildern 30A, 30B Augenstrukturen bestimmen, insbesondere Abbilder der Hornhaut mit der vorderen Homhautoberfläche 31A, 318 und der hinteren Homhautoberfläche 32A, 32B sowie Abbilder der Linse mit der vorderen Linsenoberfläche und der hinteren Linsenoberfläche. Darauf basierend werden beispielsweise Distanzen respektive Dicken bestimmt, insbesondere die Messwerte D_{A} und D_{B} der Distanzen zwischen der vorderen Homhautoberfläche 31A, 31B und der hinteren Homhautoberfläche 32A, 32B zur Bestimmung der Homhautdicke.

Wie in den Figuren 1, 2, 5, 6, 7, 8, 9 10 dargestellt ist, umfasst das erste Messsystem 6 respektive die ophthalmologische Messvorrichtung 1 ein Antriebsmodul 15 zum Rotieren des Lichtprojektionssystems 10 respektive des Lichtprojektors 11 und der Bilderfassungsmittel 12A, im wesentlichen um eine Normale zu der dem Lichtprojektor 11 zugewandten Oberfläche des Auges 3 oder zum Verschieben dieser Komponenten im Wesentlichen senkrecht zu dieser Normalen. Wie in den Figuren 7 und 8 schematisch dargestellt ist, sind das Lichtprojektionssystem 10, mit dem Lichtprojektor 11 und dem strahlumlenkenden optischen Element 123, sowie die Bilderfassungsmittel, mit dem Bildwandler 120, den strahlumlenkenden optischen Elementen 121A, 121 B und den abbildenden optischen Elementen 122A, 122B, zu diesem Zweck an einer beweglichen Trägervorrichtung 100 angebracht, welche durch das Antriebsmodul 15 angetrieben wird. Vorzugsweise sind auch die Komponenten des zweiten und dritten Messsystems 7, 8 and der Trägervorrichtung 100 angebracht. Das Antriebsmodul 15 umfasst vorzugsweise einen Rotationstreiber, beispielsweise ein Elektromotor, der die Trägervorrichtung 100 um die Strahlungsachse Z rotiert. Durch die Rotation des Lichtprojektors 11 und der Bilderfassungsmittel um die Strahlungsachse Z wird das gesamte Auge 3 vermessen. In den Ausführungsformen nach den Figuren 9 und 10 umfasst die ophthalmologische Messvorrichtung 1 einen durchbrochenen Schirmkörper 17. Die Durchbrechungen 171, 172, 173 des Schirmkörpers 17 sind jeweils so angeordnet, dass die Strahlengänge zu den Bilderfassungsmitteln 12A, 12B und zum Lichtprojektor 11 den Schirmkörper 17 ungehindert passieren können. Auf der dem Auge 3 zugewandten Seite des Schirmkörpers 17 ist ein sichtbares Muster 17', ein so genanntes Placidomuster, beispielsweise mit Kreisringen 174 angebracht, das durch die Oberfläche des Auges 3 gespiegelt wird. Auf oder neben dem Schirmkörper 17 können dem Auge 3 zugewandt auch Lichtquellen angebracht sein, beispielsweise Lichtprojektoren 16 zur Projektion von Lichtmarken 36. Eine Ansicht der dem Auge 3 zugewandten Seite des Schirmkörpers 17 mit Durchbrechungen 171, 172, 173 und dem sichtbaren Muster 17' ist in der Figur 11 dargestellt. Die Spiegelung des sichtbaren Musters 17' auf der Augenoberfläche wird durch die Bilderfassungsmittel 12B im Ansichtabbild 3A abgebildet und kann bei der Positionierung der Querschnittsabbilder 30A als künstliches Referenzmuster für die Bestimmung der relativen Position der ophthalmologischen Messvorrichtung 1 zum Auge 3 verwendet werden. Der Schirmkörper 17 ist vorzugsweise so mit dem Antriebsmodul 15 verbunden, dass er mit dem Lichtprojektor 11 und den Bilderfassungsmitteln mitbewegt wird. In einer alternativen Ausführung kann der Schirmkörper 17 auch so an der ophthalmologischen Messvorrichtung 1 angebracht sein, dass er nicht mit dem Antriebsmodul 15 gekoppelt ist, wobei die Durchbrechungen 171, 172, 173 entsprechend angepasst sind. An dieser Stelle soll angeführt werden, dass der Schirmkörper 17 auch mit den Ausführungen gemäss den Figuren 7 und 8 kombinierbar ist. Zudem kann der Schirmkörper 17 auch so angeordnet werden, dass die Bilderfassungsmittel 12A, 12B und/oder der Lichtprojektor 11 zwischen den Schirmkörper 17 und das Auge 3 zu liegen kommen, dabei sind die Bilderfassungsmittel 12A, 12B und/oder der Lichtprojektor 11 beispielsweise auf der dem Auge 3 zugewandte Seite des Schirmkörpers 17 angebracht.

Durch die Kopplung des zweiten Messsystems 8 mit dem Antriebsmodul 15 kann der Wellenfrontdetektor 19 in Bezug zum Auge 3 in verschiedene Positionen bewegt werden, beispielsweise rotatorisch im wesentlichen um eine Normale zu der Oberfläche des Auges 3 (z.B. die Sehachse) und/oder translatorisch im wesentlichen senkrecht zu dieser Normalen, so dass der Wellenfrontverlauf 190 des vom Auge 3 (insbesondere von der Retina 39) diffus zurückgestreuten Lichtbündels 180' aus mehreren Positionen erfasst und gespeichert werden kann. Da der Wellenfrontdetektor 19 typischerweise eine Anordnung mit einer begrenzten Anzahl Einzellinsen umfasst, beispielsweise ein Array von 32x32 Linsen, kann durch die Erfassung und Speicherung von Wellenfrontverläufen aus mehreren Positionen die Auflösung des Wellenfrontdetektors 19 und damit des zweiten Messsystems 8 erhöht werden. Diese verbesserte Auflösung des Wellenfrontverlaufs wird unabhängig vom ersten und dritten Messsystem 6, 7 durch eine ophthalmologische Messvorrichtung zur Bestimmung eines Wellenfrontverlaufs erreicht, welche ein Messsystem 8, mit einem Lichtprojektionssystem 18 zur Projektion eines ' Lichtbündels 180 auf das Auge 3 und mit einem Wellenfrontdetektor 19 zum Bestimmen und Speichern eines Wellenfrontverlaufs 190 des durch das Auge 3 reflektierten Lichtbündels 180', sowie ein Antriebsmodul 15 umfasst, wobei das Antriebsmodul 15 und der Wellenfrontdetektor 19 so angeordnet und gekoppelt sind, dass der Wellenfrontdetektor 19 in Bezug zum Auge 3 in verschiedene Positionen bewegbar ist. Die ophthalmologische Messvorrichtung zur Bestimmung des Wellenfrontverlaufs ist zudem eingerichtet den Wellenfrontverlauf 190 aus diesen verschiedenen Positionen zu erfassen und zu speichern. Dazu umfasst die ophthalmologische Messvorrichtung zur Bestimmung des Wellenfrontverlaufs einen Datenspeicher zur Speicherung der aus den verschiedenen Positionen erfassten Wellenfrontverläufe, - jeweils zugeordnet zu einer diesbezüglichen Positionsangabe, beispielsweise in Form eines Winkels oder in Form von Koordinaten. Vorzugsweise umfasst die ophthalmologische Messvorrichtung zur Bestimmung des Wellenfrontverlaufs zudem speziell eingerichtete Verarbeitungsmittel 13 zum Bestimmen eines Gesamtwellenverlaufs mit verbesserter Auflösung aus den gespeicherten Wellenfrontverläufen 190, beispielsweise durch (gewichtete) Mittelwertbildung.

Das Steuermodul 131 ist eingerichtet die Funktion und den Betrieb der ophthalmologischen Messvorrichtung 1 zu steuern. Das Steuermodul 131 ist insbesondere eingerichtet, das erste, zweite und/oder dritte Messsystem 6, 8, 7 das Antriebsmodul 15 und/oder die Lichtprojektoren 16 selektiv zu Aktivieren um eines oder mehrere Querschnittsabbilder 30A und jeweils zugeordnete Ansichtabbilder 3A, den Wellenfrontverlauf 190 und/oder eines oder mehrere Spiegelbilder des Musters 17` aus einer oder mehreren Positionen mit oder ohne projizierte Lichtmarken 36 zu erfassen und zu speichern. Das Steuermodul 131 ist eingerichtet, vom Benutzer beispielsweise über Bedienungselemente gewählte Operationsmodi oder Befehle entgegenzunehmen, einen dem gewählten Operationsmodus oder Befehl zugeordneten Funktionsablauf zu bestimmen, das erste, zweite und/oder dritte Messsystem 6, 7, 8 sowie das Antriebsmodul 15 entsprechend dem bestimmten Funktionsablauf zu steuern, und entsprechend dem gewählten Operationsmodus oder Befehl respektive dem zugeordneten Funktionsablauf eine oder mehrere Augencharakteristik basierend auf den erfassten QuersChnittsabbüdem 30A, Ansichtabbildem 3A, Wellenfrontverlauf 190 und/oder Spiegelbildern zu bestimmen. Grundsätzlich können die Querschnittsabbilder 30A, Ansichtabbilder 3A und Spiegelbilder gleichzeitig erfasst werden. Ein gleichzeitige Erfassung des Wellenfrontverlaufs ist möglich, wenn für die Lichtprojektoren 11, 181 Lichtquellen mit unterschiedlichen Wellenlängen verwendet und wellenlängenseleküve strahlumlenkende optische Elemente 191, 182 eingesetzt werden. Zur Erfassung von zusammengehörenden Messdaten bei gleichen Wellenlängen der Lichtprojektoren 11, 181 werden die Messsysteme zeitlich verzögert aktiviert, beispielsweise in einer definierten Reihenfolge entsprechend dem benutzerselektierten Operationsmodus respektive Befehl. In einer weiteren Ausführungsvariante mit Lichtprojektoren 11, 181 gleicher Wellenlänge wird durch Einsatz einer konjugierten Abbildung, beispielsweise mittels Lochblende, das Streulicht aus dem vorderen Augenabschnitt (Linse, Iris) ausgeblendet.

Die funktionalen Module der Verarbeitungsmittel 13 umfassen ein programmiertes Positionierungsmodul, welches die Position eines gespeicherten Querschnittsabbilds 30A relativ zum Auge 3 bestimmt. Die relative Positionierung erfolgt auf der Basis der Ansichtabbilder 3A, die den Querschnittsabbildern 30A jeweils zugeordnet sind. Die Positionsbestimmung eines Querschnittsabbilds 30A erfolgt durch Positionsbestimmung des Auges 3 relativ zur ophthalmologische Messvorrichtung 1, Dabei wird die relative Position der ophthalmologischen Messvorrichtung 1 zum Auge 3 auf Grund des Abbilds des befeuchteten Querschnittsteils 4A, der natürlichen Merkmale des Auges 3, der abgebildeten künstlichen Referenzmerkmale, beispielsweise die abgebildeten Lichtmarken 36, und/oder dem erfassten Spiegelbild des Musters 17' bestimmt.

Die funktionalen Module der Verarbeitungsmittel 13 umfassen zudem ein programmiertes Kompositionsmodul, welches mehrere erfasste und gespeicherte Querschnittsabbilder 30A relativ zueinander positioniert. Das Kompositionsmodul fügt unter Kenntnis der geometrischen Anordnung der ophthalmologischen Messvorrichtung 1 die erfassten und gespeicherten Querschnittsabbilder 30A entsprechend ihrer relativen Position zum Auge 3 respektive zueinander zu einem dreidimensionalen Abbild des Auges 3 zusammen, insbesondere zu einer dreidimensionalen (querschnittbasierten) Homhauttopografie des Auges 3.

Ein weiteres funktionales Modul, das programmierte Obemächenbestimmungsmodui, bestimmt eine approximative (musterbasierte) Homhauttopografie des Auges 3 basierend auf dem erfassten Spiegelbild des Musters 17'. Die musterbasierte Homhauttopografie wird vom Kompositionsmodul zur Vervollständigung der querschnittbasierten Homhauttopografie verwendet, wenn beispielsweise auf Grund eines Wimpernschlags oder eines anderen Schattens einzelne Querschnittsabbilder 30A teilweise oder vollständig fehlen. In einer Ausführungsvariante wird eine kombinierte Homhauttopografie durch gewichtete Mittelwertbildung aus der musterbasierten Homhauttopografie und der querschnittbasierten Homhauttopografie bestimmt, beispielsweise in Abhängigkeit der Messunsicherheit der beiden Messverfahren.

Weitere funktionale Module sind eingerichtet, geometrische und/oder optische Eigenschaften der Augenlinse 38, insbesondere deren Topografie und Brechkraft, basierend auf den Querschnittsabbildern 30A und dem Wellenfrontverlauf 190 zu bestimmen. Das programmierte Optikmodul bestimmt zunächst die Wellenaberration des Auges 3 basierend auf dem erfassten Wellenfrontverlauf 190. Das programmierte Linsenbestimmungsmodul bestimmt die Brechkraft der Augenlinse 38 basierend auf der Wellenaberration und der Homhauttopografie des Auges 3. Als Grundlage für die Bestimmung der Brechkraft kann vom Linsenbestimmungsmodul die vorher bestimmte querschnittbasierte und/oder musterbasierte Homhauttopografie des Auges 3 verwendet werden. Das Linsenbestimmungsmodul bestimmt die Geometrie der Augenlinse 38 basierend auf der Brechkraft der Augenlinse 38, der Homhauttopografie des Auges 3 und der Querschnittsabbilder 30A, 30B. Insbesondere bestimmt das Linsenbestimmungsmodul die hintere Linsenoberfläche und die vordere Linsenoberfläche der Augenlinse 38 basierend auf der Brechkraft der Augenlinse 38, der Homhauttopografie des Auges 3 und der Querschnittsabbilder 30A, 308. Vorzugsweise verwendet das Linsenbestimmungsmodul hierfür Ray-Tracing Verfahren. Mit Hilfe der Homhauttopografie verfolgt das Linsenbestimmungsmodul die Lichtstrahlen bis zur vorderen Linsenoberfläche. Eine weitere Rückverfolgung bis zur hinteren Linsenoberfläche führt das Linsenbestimmungsmodul mit Hilfe der Brechkraft der Linse durch.

Schliesslich liefert ein weiteres funktionales Modul, das Entzerrungsmodul, neben dem geometrischen Augenmodell (Homhauttopografie, Linsentopografie) als weiteres Endresultat entzerrte Querschnittsabbilder zur Darstellung auf der Anzeige 14. Insbesondere liefert das Entzerrungsmodul auf der Basis der vom Linsenbestimmungsmodul bestimmten Geometrie der Augenlinse qualitativ verbesserte, d.h. entzerrte Querschnittsabbilder der Augenlinse.

## Patentansprüche

1. Ophthalmologische Messvorrichtung (1), umfassend:
ein erstes Messsystem (6), umfassend:
ein erstes Lichtprojektionssystem (10) zur Projektion eines ersten Lichtbündels (2) durch einen Querschnittsteil (4) eines Auges (3), und
Bilderfassungsmittel zur Erfassung und Speicherung eines Querschnittsabbilds (30A) von mindestens einem Teilgebiet des durch das erste Lichtprojektionssystem (10) beleuchteten Querschnittsteils (4), aus einer ersten Position ausserhalb des ersten Lichtbündels (2), welche in Scheimpfluganordnung zum' ersten Lichtbündel (2) angeordnet sind;
ein Antriebsmodul (15); und
ein zweites Messsystem (8), umfassend:
ein zweites Lichtprojektionssystem (18) zur Projektion eines zweiten Lichtbündels (180) auf das Auge (3), und
ein Wellenlrontdetetctor (19) zum Bestimmen und Speichern eines Wellenfrontverlaufs (190), des durch das Auge (3) reflektierten
zweiten Lichtbündels (180'),
**gekennzeichnet dadurch, dass**
das Antriebsmodul (15) eingerichtet ist, den Wellenfrontdetektor (19) zum Erfassen und Speichern mehrerer Wellenfrontverläufe (190) aus verschiedenen Positionen in Bezug zum Auge (3) rotatorisch im wesentlichen um eine Normale zu einer Oberfläche des Auges (3)
in die verschiedene Positionen zu bewegen.

2. Messvorrichtung (1) nach Anspruch 1, **dadurch gekennzeichnet, dass** das erste Lichtprojektionssystem (10) und das zweite Lichtprojektionssystem (18) so angeordnet sind, dass das erste Lichtbündel (2) und das zweite Lichtbündel (180) entlang einer gemeinsamen Strahlungsachse (Z) auf das Auge (3) projiziert werden.

3. Messvorrichtung (1) nach einem der Ansprüche 1 oder 2, **dadurch gekennzeichnet, dass** die Messvorrichtung (1) ein drittes Messsystem (7) umfasst, welches einen Schirmkörper (17) mit einem sichtbaren Muster (17') umfasst, wobei der Schirmkörper (17) so angeordnet ist, dass das sichtbare Muster (17') bei der Applikation der Messvorrichtung (1) auf einer dem Auge (3) zugewandten Seite des Schirmkörpers (17) liegt, und welches dritte Messsystem (7) Bilderfassungsmittel zur Erfassung und Speicherung eines Spiegelbilds des Musters (17') auf dem Auge (3) umfasst.

4. Messvorrichtung (1) nach Anspruch 3, **dadurch gekennzeichnet, dass** der Schirmkörper (17) so eingerichtet und angeordnet ist, dass das erste Lichtbündel (2) ungehindert durch den Querschnittsteil (4) des Auges (3) projizierbar ist, dass das Querschnittsabbild (30A) ungehindert erfassbar ist, dass das zweite Lichtbündel (180) ungehindert auf das Auge (3) projizierbar ist, und dass das durch das Auge (3) reflektierte zweite Lichtbondel (180') ungehindert erfassbar ist,

5. Messvorrichtung (1) nach einem der Ansprüche 1 bis 4, **gekennzeichnet durch** ein Steuermodul (131), welches eingerichtet ist, zum wahlweisen Aktivieren von mindestens einem der Messsysteme (6, 7, 8) zur Erfassung von mindestens einem aus Wellenfrontverlauf (190), Querschnittsabbild (30A) und Spiegelbild..

6. Messvorrichtung (1) nach Anspruch 5, **dadurch gekennzeichnet, dass** das Steuermodul (131) eingerichtet ist, eines oder mehrere der Messsysteme (6, 7, 8) entsprechend einem benutzerselektierten Operationsmodus zur Erfassung von Wellenfrontverlauf (190), Spiegelbild und/oder Querschnittsabbild(ern) (30A) zu aktivieren, und dass die Messvomchtung (1) Verarbeitungsmittel (13) umfasst, welche eingerichtet sind entsprechend dem benutzersefektierten Operationsmodus mindestens eine Augencharakteristik zu bestimmen basierend auf dem Wellenfrontverlauf (190), dem Spiegeibild und/oder dem/den Querschnittsabbild(ern) (30A).

7. Messvorrichtung (1) nach Anspruch 6, **dadurch gekennzeichnet, dass** das Steuermodul (131) eingerichtet ist, mehrere der Messsysteme (6, 7, 8) entsprechend dem benutzerseleköerten Operationsmodus in einer definierten Reihenfolge nacheinander zur Erfassung von Wellenfrontvarlauf (190), Spiegelbild und/oder Querschnittaabbild(em) (30A) zu aktivieren.

8. Messvorrichtung (1) nach einem der Ansprüche 1 bis 7, **gekennzeichnet**' durch Verarbeitungsmittel (13), welche eingerichtet sind zum Bestimmen von geometrischen und/oder optischen Eigenschaften der Linse (38) des Auges (3) basierend auf mehreren Querschnittsabbildern (30A) und dem Wellenfrontverlauf (190).

9. Messvorrichtung (1) nach Anspruch 8, **dadurch gekennzeichnet, dass** die Verarbeitungsmittel eingerichtet sind, zum Bestimmen der Brechkraft der Linse (38) basierend auf den Querschnlttsabblldem (30A) und dem Wellenfrontverlauf (190).

10. Messvorrichtung (1) nach einem der Ansprüche 8 oder 9, **dadurch gekennzeichnet, dass** die Verarbeitungsmittel eingerichtet sind, zum Bestimmen einer Wellenaberration des Auges (3) basierend auf dem Wellenfrontverlauf (190), zum Bestimmen einer Homhauttopografe des Auges (3) basierend auf den Querschnittsabbildern (30A), und zum Bestimmen der Brechkraft der Linse (38) basierend auf der Wellenaberratlon und der hiomhauttopografle.

11. Messvorrichtung (1) nach einem der Ansprüche 3 bis 10, **dadurch gekennzeichnet**, däss die Messvorrichtung (1) Verarbeitungsmittel (13) umfasst, welche eingerichtet sind zum Bestimmen einer musterbasierten Hornhauttopografie des Auges (3) basierend auf dem Spiegelbild, dass die Verarbeitungsmittel eingerichtet sind, zum Bestimmen einer querschnittbasierten Hornhauttopografie des Auges (3) basierend auf mehreren Querschnotsabbildem (30A), und dass die Verarbeitungsmittel (13) eingerichtet sind, zum Bestimmen einer kombinierten Hornhauttopografie des Auges (3) basierend auf der musterbasierten Homhauttopografte und der querschnittbasierten Hornhauttopografie.

12. Messvorrichtung (1) nach einem der Ansprüche 1 bis 11, **dadurch gekennzeichnet, dass** das erste Messsystem (6) eingerichtet ist, das erste Lichtbündel (2) durch mehrere verschieden positionierte Querschnittsteile (4) des Auges (3) zu projizieren und mehrere Querschnittsabbüder (30A) von jeweils mindestens einem Teilgebiet dieser beleuchteten Querschnittsteile (4) in Scheimpfluganordnung zu erfassen und zu speichern.

13. Messvorrichtung (1) nach einem der Ansprüche 1 bis 12, **gekennzeichnet durch** ein Antriebsrnodul (15) zum Rotieren des ersten Messsystems (6) im wesentlichen um eine Normale zu der dem ersten Lichtprojektionssystem (10) zugewandten Oberfläche des Auges (3) oder zum Verschieben des ersten Messsystems (6) Im wesentlichen senkrecht zu dieser Normalen.

14. Messvorrichtung (1) nach einem der Ansprüche 1 bis 13, **gekennzeichnet durch** Verarbeitungsmittel (13), welche eingerichtet sind zum Bestimmen einer Hornhauttopografie des Auges (3) basierend auf mehreren Querschnittsabbildern (30A).

15. Messvorrichtung (1) nach einem der AnsprOche 1 bis 14, **dadurch gekennzeichnet, dass** die Messvorrlchtung (1) weitere Bilderfassungsmittel umfasst zur Erfassung eines Ansichtabbilds (3A) des Auges (3), welches Ansichtabbild (3A) mindestens eines umfasst aus einem Abbild (4A) des durch das erste Lichtprojektionssystem (10) beleuchteten Querschnittsteils (4) und einem Spiegelbild eines Musters (17') auf dem Auge (3), und zur Speicherung dieses Ansichtabbilds (3A) zugeordnet zum Querschnittsabbild (30A), und dass die Messvorrichtung (1) Verarbeitungsmittel (13) umfasst zur Positionierung des gespeicherten Querschnittsabbilds (30A) relativ zum Auge auf der Basis des zugeordnet gespeicherten Anstchtabbilds (3A).

16. Messvorrichtung (1) nach einem der Anspruche 1 bis 15, **dadurch gekennzeichnet, dass** das erste Lichtprojektionssystem (10) eingerichtet ist, das erste Lichtbandel (2) in Form eines hchtspalts zu projizieren. 6

17. Messvorrichtung (1) nach einem der Ansprüche 1 bis 16, **dadurch gekennzeichnet, dass** das erste Lichtprojektionssystem (10) und das zweite Lichtprojektionssystem (18) eine gemeinsame Lichtquelle umfassen.

18. Messvorrichtung (1) nach einem der Ansprüche 1 bis 17 **dadurch gekennzeichnet, dass** der Wellenfrontdetektor (19) als Shack-Hartmann-Sensor ausgeführt ist.

19. Messvorrichtung (1) nach Anspruch 1, **gekennzeichnet durch** einen Datenspeicher zur Speicherung der aus verschiedenen Positionen erfassten Wellenfronverläufe (190), und **durch** Verarbeitungsmittel (13) zum Bestimmen eines Gesamtwellenverlaufs aus den gespeicherten Wellenfronverlaufen (190).

## Claims

1. Ophthalmological measurement apparatus (1), comprising:
a first measurement system (6), comprising:
a first light projection system (10) for projection of a first light beam (2) through a cross-sectional part (4) of an eye (3) and
image detection means for detection and storage of a cross-sectional image (30A) of at least one subregion of the cross-sectional part (4) which is illuminated by the first light projection system (10), from a first position outside the first light beam, (2) which are arranged in the Scheimpflug configuration with respect to the first light beam (2);
a drive module (15); and
a second measurement system (8), comprising:
a second light projection system (18) for projection of a second light beam (180) onto the eye (3), and
a wavefront detector (19) for determination and storage of a wavefront profile (190) of the second light beam (180') reflected by the eye (3)
**characterized in that** the drive module (15) is designed to move the wavefront detector (19) to different positions for detecting and storing a plurality of wavefront profiles (190) from different positions with respect to the eye (3), rotationally essentially about a normal to the surface of the eye (3).

2. Measurement apparatus (1) according to Claim 1, **characterized in that** the first light projection system (10) and the second light projection system (18) are arranged such that the first light beam (2) and the second light beam (180) are projected along a common radiation axis (Z) onto the eye (3).

3. Measurement apparatus (1) according to one of Claims 1 or 2, **characterized in that** the measurement apparatus (1) comprises a third measurement system (7), which has a shielding body (17) with a visible pattern (17') with the shielding body (17) being arranged such that the visible pattern ( 17') is located, during application of the measurement apparatus (1), on a side of the shielding body (17) facing the eye (3), and which third measurement system (7) comprises image detection means for detection and storage of a mirror image of the pattern (17') on the eye (3).

4. Measurement apparatus (1) according to Claim 3, **characterized in that** the shielding body (17) is designed and arranged such that the first light beam (2) can be projected without any impediment through the cross-sectional part (4) of the eye (3), such that the cross-sectional image (30A) can be detected without any impediment, such that the second light beam (180) can be projected without any impediment onto the eye (3) and such that the second light beam (180') reflected by the eye (3) can be detected without any impediment.

5. Measurement apparatus (1) according to one of Claims 1 to 4, **characterized by** a control module (131) which is designed for selective activation of at least one of the measurement systems (6, 7, 8) for detection of at least one of the wavefront profile (190), the cross-sectional image (30A) and the mirror image.

6. Measurement apparatus (1) according to Claim S, **characterized in that** the control module (131) is designed to activate one or more of the measurement systems (6, 7, 8) in accordance with a user-selected operating mode in order to detect the wavefront profile (190), the mirror image and/or the cross-sectional image or images (30A), and **characterized in that** the measurement apparatus (1) comprises processing means (13) which are designed to determine at least one eye characteristic, corresponding to the user-selected operating mode, on the basis of the wavefront profile (190), the mirror image and/or the cross-sectional image or images (30A).

7. Measurement apparatus (1) according to Claim 6, **characterized in that** the control module (131) is designed to activate a plurality of the measurement systems (6, 7, 8), corresponding to the user-selected operating mode, in a defined sequence successively for detection of the wavefront profile (190), the mirror image and/or the cross-sectional image or images (30A).

8. Measurement apparatus (1) according to one of Claims 1 to 7, **characterized by** processing means (13) which are designed to determine geometric and/or optical characteristics of the lens (38) of the eye (3) based on the plurality of cross-sectional images (30A) and the wavefront profile (190).

9. Measurement apparatus (1) according to Claim 8, **characterized in that** the processing means are designed to determine the refractive power of the lens (38) on the basis of the cross-sectional images (30A) and the wavefront profile (190).

10. Measurement apparatus (1) according to one of Claims 8 or 9, **characterized in that** the processing means are designed to determine wave aberration of the eye (3) on the basis of the wavefront profile (190), to determine 2 cornea topography of the eye (3) based on the cross-sectional images (30A), and to determine the refractive power of the lens (38) on the basis of the wave aberration and the cornea topography.

11. Measurement apparatus (1) according to one of Claims 3 to 10, **characterized in that** the measurement apparatus (1) comprises processing means (13) which are designed to determine a pattern-based cornea topography of the eye (3) on the basis of the mirror image, **characterized in that** the processing means are designed to determine a cross-section-based cornea topography of the eye (3) on the basis of a plurality of cross-sectional images (30A), and **characterized in that** the processing means (13) are designed to determine a combined cornea topography of the eye (3) on the basis of the pattern-based cornea topography and the cross-section-based cornea topography.

12. Measurement apparatus (1) according to one of Claims 1 to 11, **characterized in that** the first measurement system (6) is designed to project the first light beam (2) through a plurality of differently positioned cross-sectional parts (4) of the eye (3), and to detect and to store a plurality of cross-sectional images (30A) of in each case at least one subregion of these illuminated cross-sectional parts (4) in the Scheimpflug configuration.

13. Measurement apparatus (1) according to one of Claims 1 to 12, **characterized by** a drive module (15) for rotation of the first measurement system (6) essentially about a normal to the surface of the eye (3) facing the first light projection system (10), or for linear movement of the first measurement system (6) essentially at right angles to this normal.

14. Measurement apparatus (1) according to one of Claims 1 to 13, **characterized by** processing means (13) which are designed to determine a cornea topography of the eye (3) on the basis of a plurality of cross-sectional images (30A).

15. Measurement apparatus (1) according to one of Claims 1 to 14, **characterized in that** the measurement apparatus (1) comprises further image detection means for detection of a view image (3A) of the eye (3), which view image (3A) comprises at least one of an image (4A) of the cross-sectional part (4) that is illuminated by the first light projection system (10) and a mirror image of a pattern (17') on the eye (3), and for storing this view image (3A) associated with the cross-sectional image (30A), and **characterized in that** the measurement apparatus (1) comprises processing means (13) for positioning of the stored cross-sectional image (30A) relative to the eye on the basis of the associated stored view image (3A).

16. Measurement apparatus (1) according to one of Claims 1 to 15, **characterized in that** the first light projection system (10) is designed to project the first light beam (2) in the form of a light slit.

17. Measurement apparatus (1) according to one of Claims 1 to 16, **characterized in that** the first light projection system (10) and the second light projection system (18) have a common light source.

18. Measurement apparatus (1) according to one of Claims 1 to 17, **characterized in that** the wavefront detector (19) is in the form of a Shack-Hartmann Sensor.

19. Measurement apparatus (1) according to Claim 1, **characterized by** a data memory for storage of the wavefront profiles (190) detected from different positions, and by processing means (13) for determining an overall wave profile from the stored wavefront profiles (190).

## Revendications

1. Dispositif de mesure ophtalmologique (1) comportant :
un premier système de mesure (6) comportant :
un premier système de projection de lumière (10) pour la projection d'un premier faisceau lumineux (2) à travers une partie en coupe transversale (4) d'un oeil (3), et
des moyens d'enregistrement d'images pour l'enregistrement et le stockage d'une image en coupe transversale (30A) d'au moins un domaine partiel de la partie en coupe transversale (4) éclairée par le premier système de projection de lumière (10), à partir d'une première position à l'extérieur du premier faisceau lumineux (2), disposés selon la disposition de Scheimpflug par rapport au premier faisceau lumineux (2) ;
un module d'entraînement (15) ; et
un deuxième système de mesure (8) comportant :
un deuxième système de projection de lumière (18) pour la projection d'un deuxième faisceau lumineux (180) sur l'oeil (3), et
un détecteur du front d'ondes (19) pour la détermination et le stockage d'une évolution du front d'ondes (190) du deuxième faisceau lumineux (180') réfléchi par l'oeil (3),
**caractérisé en ce que** le module d'entraînement (15) est installé pour déplacer le détecteur du front d'ondes (19) dans les différentes positions pour l'enregistrement et le stockage de plusieurs évolutions du front d'ondes (190) à partir de différentes positions par rapport à l'oeil (3) pour l'essentiel en rotation autour d'une normale à une surface de l'oeil (3).

2. Dispositif de mesure (1) selon la revendication 1, **caractérisé en ce que** le premier système de projection de lumière (10) et le deuxième système de projection de lumière (18) sont disposés de telle sorte que le premier faisceau lumineux (2) et le deuxième faisceau lumineux (180) sont projetés sur l'oeil (3) le long d'un axe (Z) de rayonnement commun.

3. Dispositif de mesure (1) selon une des revendications 1 ou 2, **caractérisé en ce que** le dispositif de mesure (1) comporte un troisième système de mesure (7) comportant un écran (17) avec un motif visible (17'), l'écran (17) étant disposé de telle sorte que le motif visible (17') repose sur un côté de l'écran (17) orienté vers l'oeil (3) lors de l'application du dispositif de mesure (1), et ce troisième système de mesure (7) comportant des moyens d'enregistrement d'images pour l'enregistrement et le stockage d'un reflet du motif (17') sur l'oeil (3).

4. Dispositif de mesure (1) selon la revendication 3, **caractérisé en ce que** l'écran (17) est installé et disposé de telle sorte que le premier faisceau lumineux (2) puisse être projeté librement à travers la partie en coupe transversale (4) de l'oeil (3), **en ce que** l'image en coupe transversale (30A) puisse être enregistrée librement, **en ce que** le deuxième faisceau lumineux (180) puisse être projeté librement sur l'oeil (3), et **en ce que** le deuxième faisceau lumineux (180') réfléchi par l'oeil (3) puisse être enregistré librement.

5. Dispositif de mesure (1) selon une des revendications 1 à 4, **caractérisé par** un module de commande (131) installé pour l'activation au choix d'au moins un des systèmes de mesure (6, 7, 8) pour l'enregistrement d'au moins une image en coupe (30A) et un reflet de l'évolution du front d'ondes (190),

6. Dispositif de mesure (1) selon la revendication 5, **caractérisé en ce que** le module de commande (131) est installé pour activer un ou plusieurs des systèmes de mesure (6, 7, 8) en fonction d'un mode de fonctionnement sélectionné par l'utilisateur pour l'enregistrement de l'évolution du front d'ondes (190), du reflet et/ou de l'image (des images) en coupe (30A) et **en ce que** le dispositif de mesure (1) comporte des moyens de traitement (13) installés en fonction du mode de fonctionnement sélectionné par l'utilisateur, pour déterminer au moins une caractéristique oculaire basée sur l'évolution du front d'ondes (190), le reflet et/ou l'image (les images) en coupe (30A).

7. Dispositif de mesure (1) selon la revendication 6, **caractérisé en ce que** le module de commande (131) est installé pour activer l'enregistrement successif dans un ordre défini de l'évolution du front d'ondes (190), de reflet et/ou d'image(s) en coupe (30A) de plusieurs des systèmes de mesure (6, 7, 8) en fonction du mode de fonctionnement sélectionné par l'utilisateur

8. Dispositif de mesure (1) selon une des revendications 1 à 7, **caractérisé par** des moyens de traitement (13) installés pour la détermination de caractéristiques géométriques et/ou optiques du cristallin (38) de l'oeil (3) basées sur plusieurs images en coupe (30A) et sur l'évolution du front d'ondes (190).

9. Dispositif de mesure (1) selon la revendication 8, **caractérisé en ce que** les moyens de traitement sont installés pour la détermination de la réfringence du cristallin (38) basée sur les images en coupe (30A) et sur l'évolution du front d'ondes (190).

10. Dispositif de mesure (1) selon une des revendications 8 ou 9, **caractérisé en ce que** les moyens de traitement sont installés pour la détermination d'une aberration d'ondes de l'oeil (3) basée sur l'évolution du front d'ondes (190) pour la détermination d'une topographie de la cornée de l'oeil (3) basée sur les images en coupe (30A), et pour la détermination de la réfringence du cristallin (38) basée sur l'aberration d'ondes et sur la topographie de la cornée.

11. Dispositif de mesure (1) selon une des revendications 3 à 10, **caractérisé en ce que** le dispositif de mesure (1) comporte des moyens de traitement (13) installés pour la détermination d'une topographie de la cornée de l'oeil (3) basée sur le reflet, **en ce que** les moyens de traitement sont installés pour la détermination d'une topographie de la cornée de l'oeil (3) basée sur plusieurs images en coupe (30A), et **en ce que** les moyens de traitement (13) sont installés pour la détermination d'une topographie combinée de la cornée de l'oeil (3) basée sur la topographie de la cornée basée sur la topographie de la cornée basée sur un motif et sur la topographie de la cornée basée sur les coupes.

12. Dispositif de mesure (1) selon une des revendications 1 à 11, **caractérisé en ce que** le premier système de mesure (6) est installé pour projeter le premier faisceau lumineux (2) à travers plusieurs parties en coupe transversale (4) de l'oeil (3) situées dans différentes positions et pour enregistrer et stocker plusieurs images en coupe transversale (30A) respectivement d'au moins un domaine partiel de ces parties en coupe transversale (4) éclairées selon la disposition de Scheimpflug.

13. Dispositif de mesure (1) selon une des revendications 1 à 12, **caractérisé par** un module d'entraînement (15) pour la rotation du premier système de mesure (6) pour l'essentiel autour d'une normale à la surface de l'oeil (3) orientée vers le premier système de projection de lumière (10), ou pour le déplacement du premier système de mesure (6) pour l'essentiel perpendiculairement à cette normale.

14. Dispositif de mesure (1) selon une des revendications 1 à 13, **caractérisé par** des moyens de traitement (13) installés pour la détermination d'une topographie de la cornée de l'oeil (3) basée sur plusieurs images en coupe (30A).

15. Dispositif de mesure (1) selon une des revendications 1 à 14, **caractérisé en ce que** le dispositif de mesure (1) comporte d'autres moyens d'enregistrement d'images pour l'enregistrement d'une image en élévation (3A) de l'oeil (3), cette image en élévation (3A) comprenant au moins un des éléments suivants, une image (4A) de la partie en coupe transversale (4) éclairée au moyen du premier système de projection de lumière (10) et un reflet d'un motif (17') sur l'oeil (3), et pour le stockage de cette image en élévation (3A) attribuée à l'image en coupe (30A), et **en ce que** le dispositif de mesure (1) comporte des moyens de traitement (13) pour le positionnement de l'image en coupe (30A) stockée par rapport à l'oeil basé sur l'image en élévation (3A) correspondante stockée.

16. Dispositif de mesure (1) selon une des revendications 1 à 15, **caractérisé en ce que** le premier système de projection de lumière (10) est installé pour projeter le premier faisceau lumineux (2) sous la forme d'une fente lumineuse.

17. Dispositif de mesure (1) selon une des revendications 1 à 16, **caractérisé en ce que** le premier système de projection de lumière (10) et le deuxième système de projection de lumière (18) comportent une source lumineuse commune.

18. Dispositif de mesure (1) selon une des revendications 1 à 17, **caractérisé en ce que** le détecteur du front d'ondes (19) est réalisé sous la forme d'un capteur de Shack-Hartmann.

19. Dispositif de mesure (1) selon la revendication 1, **caractérisé par** une mémoire de donnée pour le stockage des évolutions du front d'ondes (190) enregistrées à partir de différentes positions, et par des moyens de traitement (13) pour la détermination d'une évolution globale des ondes à partir des évolutions du front d'ondes (190) stockés.
